(19) Europäisches Patentamt
European Patent Office
Office européen des brevets

(11) Publication number: 0 249 219
A1

(12) EUROPEAN PATENT APPLICATION

(21) Application number: 87108395.2

(22) Date of filing: 10.06.87

(51) Int. Cl.4: C07F 15/00 , A61K 49/00

(30) Priority: 10.06.86 PL 259997

(43) Date of publication of application:
16.12.87 Bulletin 87/51

(84) Designated Contracting States:
BE CH DE FR GB IT LI NL

(71) Applicant: UNIWERSYTET WARSZAWSKI
Krakowskie Przedmiescie 26/28
Warszawa(PL)

(72) Inventor: Samochocka, Krystyna
ul. Danilowskiego 1/16
Warszawa(PL)
Inventor: Szymendera, Janusz Jozef
ul. Pruszkowska 12/160
Warszawa(PL)

(74) Representative: Ahner, Francis et al
CABINET REGIMBEAU 26, avenue Kléber
F-75116 Paris(FR)

# (54) New cis-platinum compound and a method for pharmaceutic production.

(57) The object of the invention is a pharmaceutic production, particularly for diagnosis of neoplasmas, based on the enclosed formula I. To the aqueous solution of potassium tetrachloroplatinate is added methionine by the molar relation of methionine to tetrachloroplatinate 2 to 1. The solution is mixed intensively. The aqueous solution of tetrachloroplatinate before the addition of methionine is enriched to a large concentration of chlorides, e.g. by the addition of sodium chloride and potassium chloride and pH of the mixture is brought to the value 3 to 5. Methionine is added to so prepared solution and in the course of further manufacturing process, to the complex of dichlorodimethionineplatinum is added metal salt. The components are mixed and pH of the solution is brought to the value about 6, 5. At last the solution is infiltrated through the sterile filter.

EP 0 249 219 A1

Xerox Copy Centre

# NEW CIS-PLATINUM COMPOUND AND A METHOD FOR PHARMACEUTIC PRODUCTION.

## Field of the invention

The present invention relates to a new cis-platinum compound.

The present invention also relates to a new pharmaceutical composition especially for diagnosis of neoplasm.

The present invention further relates to a method for the preparation of said new cis-platinum compound.

## Prior art

There is known as the first the metallic element compound being applied in chemotherapy of neoplasm - platinum inorganic compound, so-called "cis-platinum", being diaminodichloro-platinum, discovered in l968 year by Rosenberger and his group.

There is also known the method for production of the inorganic radioactive platinum compound, like is $^{195m}$Pt-cis-platinum.

The disadvantage of the application as radiopharmaceutic of the radioactive platinum compound, designed particularly for diagnosis of neoplasm, results first of all from the fact of a very short life of the said isotope, amounting to only just 4 days, a very short, complicated manufacturing process and also - in spite of the minimal dosis applied while diagnosis - from the high toxicity of cisplatinum.

As it was proved by the examinations on the animals, the higher dosis or equal to 2,5 mg/kg already caused the pathological effects.

There is also known the method for production of dimethionine platinum, based on that, to the aqueous solution of potassium tetrachloroplatinate is added methionine in molar relation 2 to 1 to tetrachloroplatinate and the said mixture is heated during 5 minutes, and as a result of this, chelate compound of dimethionineplatinum is obtained, in which platinum atom is coordinated by sulphur and nitrogen atoms, occured from methionine.

Dimethionineplatinum compound has not been used up till now for production of pharmaceutics. The disadvantage of the application of this known method for pharmaceutics production, results from the fact, that the said compound contains cyclic ligands.

## The object of the invention

An object of the present invention is a new cis-platinum compound, characterized in that it is a dichlorodimethionineplatinum of formula I :

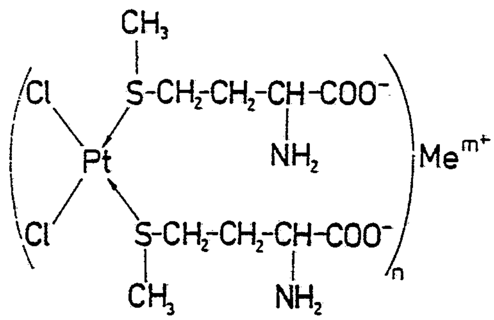

wherein Me represents a metal.

An other object of the present invention is a pharmaceutical composition especially for the diagnosis of neoplasm, characterized in that it comprises as active constituent the above new cis-platinum compound of formula I.

A further object of the present invention is a method for the preparation of compound of formula I, characterized in that an aqueous solution of potassium tetrachloroplatinate is enriched to a large concentration of chlorides,especially by the addition of sodium chloride and potassium chloride, and pH of the mixture is brought to the value 3 to 5, and than methionine is added to so obtained tetrachloroplatinate in the quantity adequate to the molar relations of methionine to tetrachloroplatinate 2 to 1, the solution is mixed intensively, to the complex of dichlorodiméthionineplatinum so obtained is added metal salt, the components are mixed and pH of the solution is brought to the value about 6,5, at last the solution is infiltrated through a sterile filter.

The essence of the invention is based on that, the change of the process engineering enables the manufacture of dichlorodimethionineplatinum, which - as it was presented formula I - contains non-cyclic ligands and - as it was proved unexpectedly by the examinations and tests - its toxicity as well as mutagenes is very low and upon tagging by metal salts, particularly by Ytterbium $^{-169}$ chloride, shows an affinity for neoplastic tissues and brings very good results in the scintigraphic examinations.

The method for pharmaceutic production according to the invention, particularly for diagnosis of neoplasmas, based on the enclosed chemical formula is that, the aqueous solution of potassium

tetrachloroplatinate is enriched to a large concentration of chlorides, e.g. by the addition of sodium chloride and potassium chloride and pH of the mixture is brought to the value 3 to 5 by the solution of 0,3 N NaOH. Next, to so obtained tetrachloroplatinate, while the continuous mixing, methionine is added in the quantity adequate to the molar relations of methionine to the tetrachloroplatinate 2 to 1. Upon the careful mixing during about 30 minutes, dichlorodimethionine platinum complex is purified particularly on the chromatographic column. To the purified complex of dichlorodimethionineplatinum, metal salt is added, mainly chloride of radioactive ytterbium $^{-169}$.

The components are mixed during 15 to 30 minutes and next, pH of the solution is brought to the value of about 6,5. At last, the solution is infiltrated through the sterile filter and pharmaceutic is ready for administration.

The advantage of pharmaceutic being manufactured according to the invention is its very low toxicity and mutagenes. A dose of $LD_{50}$ for mouse for dichlorodi-D-methionineplatinum amounts to 571 mg/kg. Some metal radionuclides, and particularly Yb-169, have a good radioactive characteristic and proper biological and physical properties. E.g. the radiation energy gamma Yb-169, which amounts to from 60 to 300 ksV as well as the time of a partial atrophy - 32 days, are the good parameters of radionuclide for the scintigraphic examinations.

The metals are making the stable complexes with dichlorodimethionineplatinum, which are purged with urine.

For example, the retention of Yb-169 in the organism of the animals under examination amounts to from 3 to 6 % after 24 hours and after 72 hours up to 1 % - what at the relatively long time of the partial atrophy - creates from one side the possibility of carrying out of the examinations at the different, longer time intervals and from the other side decreases the radiation load of the organism.

## Example

100 µl of the 3 N HCl solution was added to 7 mg of potassium tetrachloroplatinate, and next pH of the solution has been brought to the value about 4 by 0,3 N NaOH solution. Upon a careful intermixing, 5 mg D-methionine was added while the intensive mixing, and upon a careful intermixing after about 30 minutes, the complex of dichlorodi-D-methionineplatinum has been purified and 100 µl of ytterbium chloride, tagged Yb-169 of the activity 1 mCi,i.e.37 MBq was added. The reaction mixture was brought by 0,3 N NaOH to the value of pH about 5,5.

The solution obtained in such a way contains the complex of dichlorodi-D-methionineplatinum and sodium chloride as well as the complex of dichlorodi-D-methionineplatinum, tagged Yb-169.

There was ascertained practically, that the solution stored at the temperature + 4°C is stable.

For the biological examinations on the mice, 40 µl of the above mentioned solution per mouse has been taken for intravenous injection.

## Claims

1. New cis-platinum compound characterized in that it is a dichlorodimethionineplatinum of formula I :

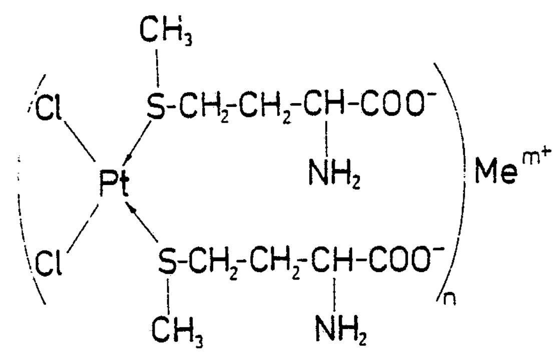

wherein Me represents a metal.

2. Pharmaceutical composition, especially for diagnosis of neoplasm characterized in that it comprises as active constituent the compound of formula I according to claim 1 especially wherein Me is a radioactive element.

3. Method for the preparation of compound of formula I according to claims 1 and 2, characterized in that an aqueous solution of potassium tetrachloroplatinate is enriched to a large concentration of chlorides, especially by the addition of sodium chloride and potassium chloride, and pH of the mixture is brought to the value 3 to 5, and than methionine is added to so obtained tetrachloroplatinate in the quantity adequate to the molar relations of methionine to tetrachloroplatinate 2 to 1, the solution is mixed intensively, to the complex of dichlorodiméthionineplatinum so obtained is added metal salt, the components are mixed and pH of the solution is brought to the value about 6,5, at last the solution is infiltrated through a sterile filter.

4. Compound of formula I according to claims 1 to 3 wherein the metal is radioactive Ytterbium $^{-169}$.

European Patent Office

# EUROPEAN SEARCH REPORT

Application number

EP 87 10 8395

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 63, no. 13, 20th December 1965, column 18249g-h, Columbus, Ohio, US; L.M. VOLSHTEIN et al.: "Reaction of methionine with trans-diamminedichloroplatinum-(II)", & ZH. NEORGAN. KHIM. 10(9), 1976-9, (1965) * Whole document * --- | 1-4 | C 07 F 15/00<br>A 61 K 49/00 |
| A | CHEMICAL ABSTRACTS, vol. 96, no. 7, 15th February 1982, page 18, abstract no. 45859k, Columbus, Ohio, US; M.F. MOGILEVKINA et al.: "Relation among the structure, properties, and antiblastic activity of complexes of platinum with methionine", & KOORD. KHIM. 1981, 7(8), 1250-4 * Whole document * --- | 1-4 | TECHNICAL FIELDS SEARCHED (Int. Cl.4)<br>C 07 F 15/00<br>A 61 K 49/02 |
| A | CHEMICAL ABSTRACTS, vol. 98, no. 22, 30th May 1983, page 695, abstract no. 190547c, Columbus, Ohio, US; D. DALIETOS et al.: "Synthesis of enantiomeric pure cis-dichloroplatinum(II) amino acid complexes", & BIOL. TRACE ELEM. RES. 1982, 4(4), 331-5 * Whole document * ----- | 1-4 | |

The present search report has been drawn up for all claims

| Place of search | Date of completion of the search | Examiner |
|---|---|---|
| THE HAGUE | 17-09-1987 | PAUWELS G.R.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document